Europäisches Patentamt

European Patent Office

Office européen des brevets

(19)

(11) Publication number: **0 263 206 B1**

(12) **EUROPEAN PATENT SPECIFICATION**

(45) Date of publication of the patent specification:
**12.12.90**

(21) Application number: **86307767.3**

(22) Date of filing: **08.10.86**

(51) Int. Cl.⁵: **C07K 13/00, C07K 3/08,
C12N 15/00, C12P 21/02,
C12N 1/20, A61K 37/02**

(54) **Enhanced bioactivity of mammalian somatotropin through selective deamidation.**

(43) Date of publication of application:
**13.04.88 Bulletin 88/15**

(45) Publication of the grant of the patent:
**12.12.90 Bulletin 90/50**

(84) Designated Contracting States:
**ES GR**

(56) References cited:
**EP-A- 0 047 600
EP-A- 0 131 843**

(73) Proprietor: **THE UPJOHN COMPANY, 301 Henrietta
Street, Kalamazoo, Michigan 49001(US)**

(72) Inventor: **Harbour, Gary C., c/o The Upjohn
Company 301 Henrietta Street, Kalamazoo
Michigan 49001(US)**
Inventor: **Hoogerheide, John G., c/o The Upjohn
Company 301 Henrietta Street, Kalamazoo
Michigan 49001(US)**
Inventor: **Garlick, Robert L., c/o The Upjohn
Company 301 Henrietta Street, Kalamazoo
Michigan 49001(US)**

(74) Representative: **Perry, Robert Edward et al, GILL
JENNINGS & EVERY 53-64 Chancery Lane, London
WC2A 1HN(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention discloses deamidated forms of mammalian somatotropins originally having an asparagine located between residues 96-101. These modified somatotropins have an increased bioactivity as compared to their native species. This invention particularly discloses a composition comprised of bovine somatotropin (bSt) compounds wherein the bSt has been modified by conversion from the native state comprising a predominant electrophoretic species having an isoelectric point (pI) of 8.2 to a bioenhanced composition of bSt molecules having a predominant electrophoretic species with a pI between 7.5 and 6.5. The preferred bovine somatotropin is that species modified at the asparagine located between amino acid residues 96-101 to an isoaspartic acid, aspartic acid or glutamic acid (see Figure 1). Chemical and recombinant genetic processes for the production of these bioenhanced compositions are also disclosed.

Bovine somatotropin is a growth hormone that has been well studied and the literature has been recently reviewed by Paladini, A.C. et al., Molecular biology of growth hormones. CRC Critical Reviews in Biochem. 15:25-56 (1983). Somatotropins were originally discovered in pituitary gland extracts from various animal species. In general, somatotropins are conserved molecules and similarities in amino acid sequences and structure are found between animals of disparate evolutionary ranking.

Growth hormones including bovine growth hormone are globular proteins comprised of a single chain of approximately 200 amino acids, having 2-3 intramolecular disulfide bonds. Accordingly, bSt has a single chain of 190-191 amino acids, globular structure with two intramolecular disulfide bonds and a molecular weight of approximately 22,000 daltons.

Natural bSt extracted from pituitary material is a heterogeneous mixture of proteins. At least six major forms of the protein have been described. The longest form is amino-terminal ala-phe bSt having 191 amino acid residues. The second form is amino terminal phe bSt having 190 amino acid residues. The third form is aminoterminal Met-bSt having 187 amino acid residues. The remaining three forms of bSt arise where an allelic variation substituting valine for leucine at position 127 in the chain is present. In addition to the above defined heterogeneity, undefined heterogeneity of bovine somatotropin has also been described. Hart, I.C. et al., The heterogeneity of bovine growth hormone. Biochem. J. 218:573-581 (1984); and in Wallace, M. and Dickson, H.B.F. A chromatographic preparation of ox growth hormone. Biochem. J. 100:593-600 (1965).

Undefined electrophoretic heterogeneity is seen when the native extracts are fractionated by anion exchange chromatography using DEAE cellulose columns. It has not been shown that the defined forms have different relative potency in bioassays. However, it has been shown that the undefined species of bSt when fractionated on ion exchange columns demonstrate varying degrees of bioactivity in rat growth models. Hart, et al. and Wallace and Dickson, supra.

This invention discloses a process for increasing the bioactivity of bSt through selective deamidation by subjecting the bSt molecules to mild heat and alkaline conditions. The general process involves the conversion of asparagine to aspartic acid or isoaspartic acid in a nonenzymatic acid or base catalyzed reaction. The rate of conversion is dependent in part upon neighboring amino acids in the polypeptide sequence.

Bovine somatotropin is known to have sites that are capable of undergoing deamidation. Conditions for in vitro deamidation and the kinetics of deamidation have been described by Lewis, U.J., et al., Kinetic study of the deamidation of growth hormone and prolactin. Biochim. Biophys. Acta, 214:498-508 (1970). It is known for bSt that as pH increases, conversion to lower pI species occurs more rapidly and that as ionic strength is increased in a buffer at pH 10 the rate of deamidation will likewise increase.

Bovine somatotropin produced by recombinant microorganisms or extracted from pituitary gland tissue has great commercial value. It increases lactation in dairy cattle and increases size and meat production in beef cattle. Administration to cattle is a problem. It is estimated that upwards to 100 mg per animal per day will be needed to effect commercially acceptable improvements in production. Such a dosage will require efficient methods of administration. Improvements in the potency of bSt such as described in this invention will be of benefit because of resulting reductions in the amount of drug administered to each animal per day.

## SUMMARY OF THE INVENTION

This invention relates to the enhancement of bioactivity for mammalian somatotropins including bSt. It is disclosed herein that the bioactivity of mammalian somatotropins can be enhanced by selective diamidation. More specifically, this invention discloses that milk production and growth rates in animals are enhanced by partially deamidating the bSt molecules prior to administration.

The chemical modification described in this invention results in an increase in the proportion of electrophoretic species of bSt having an approximate pI of 7.0. In order to increase the presence of these species, the native forms are incompletely deamidated using mild alkaline conditions and heat. The selectively deamidated species of bSt are capable of increasing growth rate, milk production, and numbers of mammary parenchymal cells in animals at a greater rate than an identical amount of a native pituitary bSt or recombinant bSt. In addition to disclosing the compounds, processes for making and using the deamidated bSt are described.

Specifically, this invention describes a composition comprised of bovine somatotropin-like compounds having a mixture of electrophoretic species wherein the dominant species have isoelectric points of between 7.5 to 6.5.

This invention also embraces a partially deamidated species of mammalian somatotropins wherein the asparagine located between amino acid residues 96-101 is converted to isoaspartic acid or aspartic acid.

Although not all mammalian somatotropins contain an asparagine at the residues 96-101 of their amino acid sequence, porcine and human growth hormones in addition to bSt do contain a single asparagine at the appropriate location. Accordingly, those native mammalian somatotropins having an asparagine at this region, which corresponds to a β turn, are embraced by this invention. In addition to the native species, analogs of bSt and other mammalian somatotropins having an asparagine in a region corresponding to the 96-101 residues of the native somatotropins are embraced by this invention. Such analogs include somatotropin-like compounds which embrace all proteins having sufficient similarity in amino acid sequence to elicit a growth response in the hypophysectomized rat assay or in the mammal from which it was derived.

More specifically and preferred are those species of bSt-like compounds wherein the asparagine located between amino acid residues 96-101 is converted to isoaspartic acid or aspartic acid.

Due to the polymorphism of somatotropins, the position numbers of amino residues of the various somatotropins may differ. The term "native mammalian somatotropin" embraces these naturally occurring species. In this document figure 1 illustrates the specific region of one species of bSt that corresponds to the preferred modification site of this invention. The numbering for other somatotropins may differ where analogs are involved and it is helpful to note that the asparagine of interest appears within a β turn which is calculated according to Chou and Fasman, J. Mol. Biol. 115:135 (1977) and Ann. Rev. Biochem. 47:251 (1978). Using the general region enumerated as 96-101 of the native sequences and the knowledge that the region is a β turn, those of ordinary skill in the art can readily locate corresponding amino acid sequences capable of undergoing deamidation in alternative native mammalian somatotropins or their analogs to achieve the desired enhanced bioactivity.

Both chemical and genetic modifications of this region are embraced by this invention. Chemical deamidation results in both aspartic acid and isoaspartic acid being formed in place of the naturally occurring asparagine. The preferred chemical process for increasing the proportion of electrophoretic species of bovine somatotropin having isoelectric points between 7.5 and 6.5 involves treating recombinant or pituitary bovine somatotropin with a mild alkaline treatment or heat or both. In addition a process is disclosed for further increasing the proportion of desired electrophoretic species by removing those species having an isoelectric point outside of the range of 7.5 to 6.5 using chromatographic or electrophoretic techniques or a combination thereof. The preferred method of isolation utilizes reversed phase high performance liquid chromatography.

The preferred genetic modificatons rely upon single site specific mutation methods for insertion of negatively charged amino acids in replacement or addition to the naturally occurring asparagine. The charged amino acids include aspartic acid and glutamic acid.

A process is also disclosed for enhancing the growth rate of animals by treatment with an effective amount of bovine somatotropin that has been modified to contain a preponderance of electrophoretic species having isoelectric points of between 7.5 to 6.5. In addition this process of increasing the growth rate of animals is more specifically disclosed for use in bovine-type animals.

A method of use is also disclosed for increasing the quantity of mammary parenchymal cells in a female ruminant that comprises treatment of the ruminant at a developmental stage of between at about the onset of puberty and the first parturition with an effective amount of bovine somatotropin that has been modified to contain a preponderance of electrophoretic species having isoelectric points of between 7.5 to 6.5. In addition the above method is more particularly described wherein the modified bovine somatotropin is administered between at about the onset of puberty until the first conception.

A method of use is also disclosed for increasing milk production in female ruminants comprising the administration of an effective amount of bovine somatotropin that has been modified to contain a preponderance of electrophoretic species having isoelectric points of between 7.5 to 6.5. Lastly this method is described more particularly wherein the ruminant is a dairy cow.

The terms "deamidated or deamidation" refer to chemically or recombinant genetically introduced modifications to a somatotropin which result in the conversion of an asparagine to an isoaspartic acid, aspartic acid, or glutamic acid. Chemically introduced modifications result in isoaspartic acid or aspartic acid in place of asparagine. Recombinant genetically introduced modifications result in either an aspartic acid or glutamic acid residue in place of asparagine. The disclosed chemical treatments initially deamidate the preferred asparagine residue but will with time deamidate any available asparagine or glutamine. The conversion of an asparagine residue to a negatively charged residue will alter the somatotropin's profile on an isoelectric focusing gel and can be monitored using isoelectric focusing methods. The phrase "partial or selective deamidation" refers to incompletely deamidated somatotropin-like proteins having an isoelectric point between 7.5 and 6.5.

The phrase "closest-related native somatotropin" refers to the naturally-occurring form of mammalian somatotropin which when compared to a specific somatotropin-like protein is more closely identical in amino acid sequence than any other naturally-occurring form of mammalian somatotropin.

The phrase "somatotropin-like protein" refers to both native forms of somatotropins and to analogs of native somatotropins provided that the analogs have sufficient protein identity with their parent compounds to demonstrate bioactivity as either a growth promoter or as a stimulant for milk production.

The phrase "mammalian somatotropin" refers to somatotropins originating from mammals and includes somatotropins derived from either natural sources, e.g., pituitary gland tissue or from microorganisms transformed by recombinant genetics to produce a naturally-occurring form of somatotropin. When a specific mammalian source is named such as a bovine somatotropin or a somatotropin of bovine origin, the somatotropin includes those derived from either natural sources or from transformed microorganisms.

The term "microorganism" refers to both single cellular prokaryote and eukaryote organisms such as bacteria, yeast, actinomycetes and single cells from higher order plants or animals when being grown in cell culture.

The term "native" refers to naturally-occurring forms of somatotropins which may have been derived from either natural sources, e.g., pituitary gland tissue or from microorganisms transformed by recombinant genetics to produce a naturally-occurring form of somatotropin.

The term "vector" includes both cloning plasmids and plasmids which are capable of directing the expression of a somatotropin by virtue of the cDNA encoding the somatotropin being operatively linked to a promoter capable of being recognized by an microorganism.

## Detailed Description

The mammalian somatotropins are very similar in amino acid sequence and physical structure. Although the processes described below are directed towards the deamidation of bSt, the processes are equally applicable to any mammalian somatotropin having the requisite asparagine residue available for deamidation.

The ionic species in a typical sample of bSt from a pituitary gland or recombinant microorganism range from isoelectric points of 9.0-5.5. The chemically modified bSt is also a mixture of species; however, the species having isoelectric points inside the bioenhanced region of 7.5 to 6.5 predominate.

The basis of this invention resides in the determination that the natural electrophoretic heterogeneity of bSt can be altered by deamidation to yield an increased proportion of species having a pI of approximately 7.0 to achieve an increased biological effect. Hart, et al. and Wallace and Dickson, supra. Although, the prior literature indicates that variations in bioactivity exist between electrophoretic species of bSt, the most acidic of the naturally-occurring forms of bSt was shown to have the most biological potency. This invention demonstrates that after mild chemical treatment, the ionic species having the greatest biological potency reside not with the species having the most acidic isoelectric point but rather in the middle range of species having a pI of between 6.5 and 7.0. Moreover the literature does not suggest any correlation between deamidation and the variability of bioactivity found in heterogeneous extracts of bSt. In fact, the cause of the heterogeneity was not speculated upon in the above cited references and it is not known whether undefined heterogeneity exhibiting biological variation is due to genetic variability, to in vivo post-translational modification, to differences in phosphorylation (Liberti, J.P. et al., Biochem. and Biophys. Res. Comm. 128:713-720, 1985) or to artifacts of isolation. The presence of minor differences in the amino acid sequences of bSt, due for example to polymorphism, is not relevant to this invention. The described modifications are able to enhance all bioactive forms of bSt whether it is obtained from pituitary glands or from recombinant microorganisms. The preferred form of bSt for chemical deamidation is a species produced by a recombinant microorganism because it is less heterogeneous than pituitary extracted bSt and more economical to produce. The most preferred deamidated species of bSt having an approximate pI of 7.0 has been selectively modified at the asparagine located between residues 96-101 to isoaspartic acid.

Analogs of bSt are known (see European patent applications 75,444 and 103,395). Such analogs are also capable of being modified to enhance bioactivity and are considered a part of this invention. Moreover, it is possible to selectively mutate any of the bSt analogs or native species to accommodate either a glutamic acid or aspartic acid within the 96-101 region of the bSt molecule. See, for example, Nucleic Acid Res. 10(20):6487 (1982).

The processes for chemically modifying somatotropin-like proteins include acid treatment or base treatment. The treatment described below partially deamidates bSt and is preferred. Efficient deamidation is achieved by alkaline treatment and elevated temperatures. A pH range of 7.5 to 11 and a temperature range of 20° to 50°C is generally effective. Ionic strength is also a factor and a range of 0.01 to 2.0 dependent on the particular buffer and salts being present is effective. Without control of the process, as described below, the predominant bSt species will have a pI of 6.0 or below. The duration of chemical treatment varies according to temperature and pH. At elevated temperatures and at the pH extremes, deamidation occurs more rapidly than at low temperatures and neutral pH. Isoelectric focusing or reversed phase high performance liquid chromatography procedures can be used to monitor the process.

The most acidic species of bSt (pI 5.0) is the least active species in relative potency. The most potent species are only partially deamidated and fall within the 7.5-6.5 region. To optimize the production of

4

those bioenhanced forms having a pI of around 7.0, mild deamidating methods must be employed. For example, recombinant bSt can be suspended in alkaline buffers such as Tris-HCl, sodium borate, sodium carbonate or sodium phosphate at an ionic strength of 0.01 to 0.2, and heated to 30°-50°C noting that rate of deamidation will increase rapidly with increased temperature. These mild conditions permit partial deamidation to occur slowly over a 4-5 day period. The concentration of recombinant bSt ranges from 1.0 to 10 mg/ml.

After the selective deamidation has been completed, chromatographic or electrophoretic techniques are optionally used to purify the mixture of ionic species. The aim of the purification is to increase the proportion of ionic species of bSt having an isoelectric point around 7.0 and increase the bioactivity of the final product. Techniques for isolation and purification include preparative isoelectric focusing and ion exchange chromatography with anion exchange chromatography being a preferred technique. The most preferred method of separation is reversed phase high performance liquid chromatography. The preferred species of deamidated bSt has a different retention time from the native species allowing for easy separation and purification.

The high relative potency of the deamidated bSt is readily determined using hypophysectomized rats. Evans, H.M. and Long J.A., The effect of the anterior lobe of the hypophysis administered intraperitoneally upon growth, and the maturity and oestrus cycles of the rat, Anat. Rec. 21:61, 1921. Relative increases in total body weight are recorded using pituitary bSt, recombinant bSt [rbSt] and various fractions of deamidated rbSt. Results in rats using ionic species of deamidated rbSt having a pI of approximately 7.0 demonstrate a 2-3 times greater potency than native undeamidated bSt.

Administration into dairy cattle is according to known methods using any route effective to deliver the required dosage to the animals circulatory system. Modes of administration include oral, intramuscular injections, subcutaneous injections and the use of timed-release implants. The preferred mode of administration is by subcutaneous injection using a timed-release implant. Appropriate vehicles for injections include physiologically compatible buffers such as sodium bicarbonate, sodium phosphate, or ammonium phosphate solutions. Timed-release implants are known in the art, e.g., U. S. Pat. 4,333,919.

The effective dosage range is from 1.0 to 200 milligrams per animal per day. The greater the amount of bSt given, the greater the resulting increase in growth, lactation or numbers of mammary parenchymal cells. Most preferably, the dosage range is from 10 to 50 milligrams per day.

Mammalian growth hormones are very similar in their amino acid sequences and hormones originating from one animal source can enhance the growth of other unrelated species of animals. For purposes of increasing growth rate of animals, deamidated bSt can be used to produce increased growth in the same animal species in which native bSt has been shown to have growth-related bioactivity such as bovines, sheep, rats, salmon and chickens. The preferred animals are bovine used for beef cattle such as bulls, heifers or steers.

Beef cattle are slaughtered just prior to reaching full maturity and size. Deamidated bSt can be used to produce increased growth rates in beef cattle by administration any time between weaning until slaughter. Deamidated bSt is administered to beef cattle for a minimum of 30 days and for a maximum of 450 days depending upon desired time of slaughter. Animals used for veal are typically slaughtered at approximately 6 months of age and 10 to 30 mg/day of deamidated bSt is administered up until the age of slaughter to effectuate desired increases in growth rate.

For purposes of increasing lactation in bovines, particularly dairy cows, deamidated bSt is administered between 30 and 90 days post-partum and continued for up to 300 days. Deamidated bSt will also increase lactation in other commercial milk-producing animals such as goats or sheep.

For purposes of increasing the number of mammary parenchymal cells in female ruminants, particularly dairy heifers, the methods described in U.S. Patent 4,521,409 are incorporated by reference herein. Briefly the ruminants which include dairy heifers, sheep and goats among others are treated with the deamidated bSt sometime around the onset of puberty. Treatment is continued until first conception or parturition. More particularly administration of deamidated bSt in dairy heifers can be begun within 30 days of the expected onset of puberty and continued up until 100 days after the onset of puberty.

Puberty is not determinable by visual inspection. Puberty is defined by the period beginning with the initiation of the sexual process and lasting up until the maturation of the sexual organs. The precise time of administration will depend upon the particular breed of animal, its nutritional status and management practices.

In this section and throughout this document, parameters described by a range such as 1 to 5 or 1-5 milligrams, days, hours, degrees, or pH units are meant to be inclusive unless otherwise stated.

Examples

Example 1. Recombinant bSt [rbSt].

Recombinant bSt is known and can be produced by a number of published procedures such as described in Schoner, B.E., et al., Role of messenger RNA translational efficiency in bovine growth hormone expression in Escherichia coli. PNAS, USA, 81:5403-5407 (1984); Seeburg, P.H. Efficient bacterial expression of bovine and porcine growth hormones. DNA, 2:37-45 (1983) and European patent application 75,444. The choice of rbSt species is not relevant to this invention. N-terminal heterogeneity is known to exist for bSt. Two species exemplified herein are the amino-terminal ala-phe- and phe- forms of rbSt. The chemical deamidation and biological tests are exemplified with ala-phe rbSt. Forms of rbSt that are expressed by E. coli may have a methionine residue in place at the N-terminal portion of the protein. This additional residue does not influence the nature of this invention and its presence is optional.

Example 2. The physical deamidation of asparagine at amino acid residue 99 of rbSt.

These are preferred deamidation conditions which optimize deamidation of the asparagine at residue 99 of ala-phe-rbSt.

A. Purified recombinant bovine somatotropin, approximately 300 mg, is incubated for 168 hours in 16.5 ml 0.15M sodium carbonate, pH 10.0 at 37°C. After incubation, the rbSt is dialyzed versus deionized water and lyophilized. The rbSt component containing isoaspartic acid as the major amino acid at position 99 in the sequence, is isolated by reversed phase HPLC. J. Chem. Soc. Perkin Trans., 1:2311-2318 (1984). The described mechanism should not be construed as a limitation of the disclosed invention.

An alternative process for partially deamidating bSt uses a buffer of 100 mM sodium phosphate at pH 9.45 having a concentration of bSt of 1-2 mg/ml which is then heated at 37° for 96 hours. This material is then dialyzed against dilute NaOH, followed by lyophilization.

B. For analytical HPLC, approximately 50 µg of rbSt is applied to a 0.41 x 25 cm Vydac C-4 reversed phase HPLC column at room temperature, using a linear solvent gradient containing 0.1% trifluoroacetic acid as the A solvent and 100% acetonitrile as the B solvent. The flow rate is constant at 1 ml per minute. The gradient conditions are given below.

At the beginning of chromatography, the concentration of acetonitrile is 35%. The concentration of solvent B increases to 50% over 30 minutes and then is maintained at 50% for another 15 minutes, after which the B solvent is increased to 100% over 5 minutes. Using these solvent elution conditions, the rbSt component which is modified at asparagine residue 99, elutes approximately 2 minutes earlier than native rbSt. The relative retention time of the modified rbSt is approximately 0.94, when compared to the retention time of native rbSt.

To prepare the modified and native rbSt for the rat growth bioassays, reversed phase HPLC is conducted using two 0.41 x 25 cm Vydac C-4 columns connected in series, and the same solvent gradient as is used in analytical studies. For preparative HPLC, up to 10 mg rbSt is applied to the columns for each run. In preparative chromatography, the relative retention time for the modified rbSt component, compared to native rbSt, is approximately 0.90.

The fractions containing deamidated recombinant bSt having a pI of 7.5 to 6.5 are then collected, made 6 M in urea, titrated to pH 10-10.5 with ammonium hydroxide, dialyzed against dilute NaOH and freeze dried. For injections, the material is then reconstituted in a buffer of 0.03 M NaHCO₃, and 0.15 M NaCl at pH 9.5.

Example 3. Introduction of nucleotide changes at positions "98" and "99" in bSt using recombinant genetics.

Changes in the DNA sequence of rbSt are made by site-directed mutagenesis using the methods of Zoller and Smith (Nucl. Acid Res. 10:6487-6500,1982 and Methods in Enzymology 100:468-500, 1983). In brief, a segment of DNA from the rbSt gene is cloned into M13mp phage vectors and used to infect E. coli. Single stranded DNA can be isolated from the M13 phage virions, and hybridized with a synthetic oligomer which contains the desired base changes. A double stranded region is isolated after primer extension by digestion with restriction endonucleases and cloned into a "transition vector". Clones containing the transition vector are then sequenced to verify the desired change. The transition vector provides restriction sites which allow the reconstitution of the altered rbSt gene and its introduction into an expression vector.

Example 3A is based on the modified rbSt gene in the expression vector pBGH33-4, as described in the Genentech European Patent application 75444 and Great Britain Pat. No. 2,147,902B. This gene has been modified in its N-terminal DNA sequence to enhance expression of the rbSt protein. This example should not be construed as a limitation upon the disclosed invention. The use of pBGH33-4 to exemplify site specific mutations of rbSt at codon 98 is a matter of convenience. Alternative vectors containing equally useful cDNA encoding for bSt have been described. It should be noted that pBGH33-4 contains the phe-rbSt gene and amino acid references to position 98 correspond to position 99 in the ala-phe-rbSt used for chemical diamidation and biological testing.

Example 3B discloses a convenient method for inserting an alanine into the codon at position 1 of phe-rbSt cDNA.

Unless otherwise indicated, methodology presented below relating to procedures for carrying out recombinant genetic manipulations which include enzymatic reaction conditions, DNA isolation and purification, and culturing of transformed bacterial colonies are according to Maniatis et al., Molecular Cloning: A Laboratory Manual. Cold Spring Harbor Laboratory, 1982.

Conventions used to represent plasmids and fragments in charts 1-8, though unique to this application, are meant to be synonymous with conventional representations of plasmids and their fragments. Unlike the conventional circular figures, the single line figures on the charts represent both circular and linear double-stranded DNA with initiation or transcription occurring from left to right (5′ to 3′) where indicated. Asterisks (*) represent the bridging of nucleotides to complete the circular form of the plasmids. Fragments do not have asterisk marks because they are linear pieces of double-stranded DNA. Endonuclease restriction sites are indicated above the line. Gene markers are indicated below the line. The relative spacing between markers do not indicate actual distances but are only meant to indicate their relative positions on the illustrated DNA sequence.

Example 3A. The introduction of glutamic or aspartic acid at position "99" of phe-rbSt.

A. Construction of the Transition Vector

The purpose of the transition vector is to provide a vector which has manipulatable restriction sites for reconstruction of the intact rbSt gene after a sub-segment has been mutagenized. Specifically, this example takes advantage of a PstI restriction site in the rbSt cDNA sequence located upstream of the 98th codon. This PstI site is unique in the gene but is not unique for the Genentech vector pBGH33-4. To modify codon 98, a PstI/BamHI restriction fragment encompassing this sequence is cloned into the M13mp19 vector (Messing, Methods in Enzymology 101:20-78,1983 and Yanisck-Perron et al., Gene 33:103-119, 1985). These M13mp vectors are commercially available (International Biotechnology, Inc., New Haven, Connecticut, USA). After mutagenesis of the PstI/BamHI rbSt sub-segment, the rbSt gene is cloned into a transition vector in which the PstI and BamHI restriction sites of the original rbSt gene are unique.

The construction of pUC19(d) - Chart 1.

This vector has no PstI sites. The pUC19 vector (International Biotechnology, Inc.), contains a single PstI site located in a polylinker (Yanisch-Perron et al., 1985 supra). The site lies between the HinII and HindIII restriction sites. The PstI site is deleted by cutting purified DNA at the HinII and HindIII sites, filling the 5′ overhang of the HindIII site with PolI Klenow (PolIk), purifying the large cut vector fragment by agarose gel electrophoresis and electroelution, ligating with T4 DNA ligase and ATP, and transforming competent cells selecting for resistance to ampicillin. Verification of the presence of pUC19(d) in a clone is obtained by isolating DNA from several clones using the method of Birnboim and Doly (Nucl. Acid Res. 7:1513-1523,1979). The isolated DNA is then analyzed by restriction digestions for vectors which are not cut by HindII, HinII, or PstI and are cut once by XbaI and BamHI. These procedures are described in detail in Maniatis et al., Molecular Cloning: A laboratory Manual. Cold Spring Harbor Laboratory, 1982. The resultant vector is designated as pUC19(d).

The construction of pUC19(d)-bSt - Chart 2.

The entire rbSt gene is isolated from the vector pBGH33-4 on an XbaI, BamHI restriction fragment 1 (0.9 kb) and cloned into the complementary XbaI, BamHI restriction sites of pUC19(d). To insure efficient cloning, fragment 1 (0.9 kb) is isolated from pBGH33-4, and fragment 2 (2.7 kb) is isolated from pUC19(d) after the individual vectors are digested with XbaI and BamHI. The fragments are cut from a gel and isolated by electroelution. The steps involved are the same as those described for the construction of pUC19(d). The resultant vector is designated as pUC19(d)-bSt.

Construction of pM13-bSt′ - Chart 3.

To obtain pM13-bSt′, a sub-fragment of the rbSt gene encoding the asparagine residue at position 98, is cloned into the M13mp19 phage vector. The M13 vectors permit the isolation of single stranded closed circular DNA from phage virions isolated from infected bacteria. This single stranded DNA is then hybridized with synthetic oligomers that have been altered for codon 98 forming a limited heteroduplex.

A M13 clone containing pM13-bSt′ is constructed by isolating the PstI/BamHI fragment 3 (0.7 kb) from the pBGH33-4 and cloning into the corresponding PstI/BamHI restriction sites of M13mp19 vector. The M13mp19 cloning protocol has been described by Messing in Methods in Enzymology 101:20-78, (1983). In brief, RF (replicative form) DNA is isolated from M13Mp19 infected cells. The isolated DNA is subjected to restriction digestion by PstI and BamHI endonucleases. The large fragment (about 7.2 kb) is purified and ligated to fragment 3 to yield pM13-bSt′. E. coli cells are transfected and individual plaques are isolated. The RF DNA is isolated again and the insertion of the fragment is confirmed by analytical digestions with restriction endonucleases. The PstI restriction site for rbSt cDNA corresponds to amino acids 89 and 90 of rbSt. The target codon, 98, is located within the PstI, BamHI fragment.

B. Modification of position 98

Design of DNA Oligomers.

Two synthetic oligonucleotides, 39 nucleotides in length are shown in figure 2. These oligonucleotides vary in their sequences for the codon coding for the native asparagine residue at position 98. The two oligomers will result in amino acid changes to either aspartic acid (asp), or glutamic acid (glu) at position 98. Nine nucleotides are present after the modification at position 98 to ensure the availability of 3′ end which can be extended by any of several polymerases (i.e., AMV reverse transcriptase, T4 DNA polymerase or Pol I Klenow fragment). The oligomers are made using an Applied Biosystems nucleotide synthesizer.

Preparation of Single Stranded DNA.

Preparation of single strand DNA from the M13 infected E. coli clones has been described by Messing (supra). The preferred E. coli host is JM101 also described by Messing. This host has been modified by the introduction of a the dam-13::Tn9 by P1 transduction (Miller, Experiments in Molecular Genetics, Cold Spring Harbor Laboratory, 1972) and selection for chloramphenicol resistance. An appropriate strain containing the dam-13 allele is publicly available from the E. coli Genetic Stock Center, Yale University, New Haven, Conn. USA. The dam allele is used to prevent methylation of the single stranded DNA which in turn prevents preferred correction of the altered codon (Pukkila et al. Genetics 104:571-582., 1983).

Hybridization

Hybridization conditions are described by Zoller and Smith (Methods in Enzymology 100:468-500, 1983). An oligomer with the desired codon change at position 98 is hybridized in a 20 to 30 molar excess to its complement with 1 pmole of the single stranded DNA molecules by heating at 65°C and allowing the mixture to cool to room temperature.

Primer Extension

After hybridization, the 3′ end of the oligomer is used as a primer for extension by DNA PolIk. The extension is performed in the presence of $(7.4 \times 10^5 \text{ Bq})$ $(20\mu\text{Ci})$ (alpha-$^{32}$P)dATP and 5mM each of dCTP, dGTP, and dTTP. The mixture is incubated at room temperature for 10 minutes, after which an excess of non-radioactive dATP is added. This increases the size of the heteroduplex and extends the double stranded region of the molecules to include the BamHI restriction sequence, thus creating a cleavable BamHI restriction site.

Isolation and Cloning of the Heteroduplex into pUC19(d)-bSt*-Chart 4.

Primer extended molecules are subjected to restriction digestions with PstI and BamHI. This will generate a heteroduplex of approximately 700 bp which can be visualized by autoradiography after the reaction mixture is run on a non-denaturing acrylamide gel with appropriate sizing markers (Maniatis et al., supra). The band is cut out as a gel slice and the fragment is eluted from the gel.

This DNA which contains the desired codon change in one DNA strand is then ligated into the PstI, BamHI restriction sites of the transition vector pUC19(d)-bSt. The pUC19(d)-bSt vector is digested with the PstI and BamHI restriction enzymes to yield fragment 6 (3.0 kb) which is purified by gel electrophoresis and electroelution. This removes the normal coding sequence at position 98. Fragment 6 is then ligated to the heteroduplex fragment and transformed into competent cells selecting for resistance to ampicillin. The isolation of the heteroduplex fragment eliminates the need for alkaline sucrose gradients in the Zoller and Smith (supra, 1983) protocol and insures the formation of a double stranded covalently closed molecule.

Identification of the change at Position 98.

Individual transformed cells are twice streaked from individual colonies for purification of cloned DNA. One colony for every originally transformed cell is selected and vector DNA is prepared by the protocol of Birnboim and Doly (supra). The double stranded vector DNA is sequenced by the dideoxy method using a synthesized primer to the rbSt sequence 5′ to the PstI site and position 98 (Sanger et al., Proc. Natl. Acad. Sci. USA 74:5463-5467, 1977; Zagursky et al., Gene Anal. Techn. 2:89-94, 1985). The theoretical yield of vectors having the desired changes will be 50%.

C. Construction of Expression Vector pBGH33-4-"Glu98" - Chart 5.

The modified rbSt gene can now be recloned as an XbaI, BamHI fragment from the transition vector, pUC19(d)-bSt*, into the Genentech expression vector pBGH33-4. The transition vector is digested with the restriction enzymes XbaI and BamHI and fragment 7 (1.0 kb) is obtained. Fragment 7, which contains the entire coding sequence for rbSt, is isolated. The rbSt expression vector pBGH33-4 is digested with the same enzymes and the vector fragment 8 (4.0 kb) is isolated. The two fragments are ligated and transformed into competent cells. The pBGH33-4 vector fragment provides a ribosomal binding site and the tryptophan promoter for the expression of the modified rbSt gene. The modified rbSt gene expression in this vector is inducible by methods known in the art. Purification of rbSt from transformed microorganisms is also known in the art.

Example 3B. The introduction of glutamic or aspartic acid into position 99 of ala-phe-rbSt.

Following the procedures outlined in Example 3A, it is possible to genetically alter the asparagine at position 99 of a ala-phe- form of rbSt. To obtain the ala-phe- form one can insert a codon encoding alanine between the methionine and phenylalanine codon at the N-termini of the rbSt cDNA of pBGH 33-4. The following protocol describes changing the coding sequence of the rbSt gene described by Genentech to code for a met- ala-phe-pro rbSt protein molecule. This modification can be made either before or after the changes previously described for the codon at position "98" in the rbSt molecule.
General Strategy.

The plasmid pBGH33-4 contains an XbaI restriction site located between the ribosomal binding site and the ATG initiation codon of the rbSt gene. Approximately 72 nucleotides downstream of this site there is a PvuII restriction site. A synthetic double stranded oligomer can be inserted between these two restriction sites which contains the additional ala codon at the second amino acid position. However, the PvuII restriction site is not unique for the gene. This problem can be circumvented by constructing a vector containing only that portion of the rbSt gene which is to be modified and in which this PvuII site is unique. The front end of the gene is modified and an intact rbSt gene is reconstructed.
The construction of pUC8-bSt' - Chart 6.

Plasmid pUC8-bSt' is constructed by digesting the DNA of pBGH33-4 with EcoRI and PstI restriction endonucleases. Fragment 9 (550 bp) is cut from an agarose gel and electro-eluted. This fragment contains the trp promoter, a ribosomal binding site and the front half of the rbSt gene. The pUC8 vector (commercially available from Bethesda Research Laboratories, Gaithersburg, Maryland, USA) is digested with EcoRI and PstI and the 2.7 kb vector fragment 10 is isolated. The two purified DNA molecules are ligated and transformed into competent E. coli cells. Cells containing the vectors are selected by resistance to ampicillin. DNA is isolated from the individual colonies by the Birnboim and Doly protocol and the vector construction is conformed by analytical restriction analysis. The resultant vector is designated pUC8-bSt'. The vector is unique for the XbaI and PvuII restriction sites in the fragment cloned from pBGH33-4.
The construction of pUC8-bSt'ala - Chart 7.

Plasmid pUC8-bSt' is isolated and digested with XbaI and PvuII restriction endonucleases. Fragment 11 (2.7 kb) is isolated. This removes the sequence for the first 23 amino acids of rbSt.
The following synthetic oligomers are made:
5'-CTAGAATG   GTCTTCCCAGCTATGTCTCTATCTGGTCTATTCGCTAACGCTGTTCTTCGTGCTCAG-CAT TTCATCAG-3'
5'-CTGATGAAGATGCTGAGCACGAAGAACA       GCGTTCGCAATAGACCAGATAGAGACATAGCTG-GGAA GACCATT-3'
These two synthetic oligomers are hybridized together, producing a double stranded DNA fragment 12 which incorporates the recommended sequence changes to permit efficient expression of rbSt in E. coli, and has a codon for alanine inserted at the second position as shown by the underlined sequence. This DNA molecule also has the 5' complementary overhang for XbaI and the blunt end site for PvuII. The oligonucleotide is ligated into the isolated pUC8-bSt' vector, and transformed into competent E. coli cells. Confirmation of the construction is obtained by sequencing the region. The new vector is designated pUC8-bSt'-ala.
Construction of the ala rbSt gene - pUC19(d)bSt-ala - Chart 8.

DNA of the transition vector pUC19(d)bSt (Chart 2) is digested with XbaI and PstI. The large vector fragment 13 (2.7 kb) is isolated. Next, pUC8-bSt'ala (Chart 7) is digested with XbaI and PstI restriction endonucleases and a 300 bp fragment 14 which encompasses the front half of the rbSt gene containing the ala codon insertion is isolated. The vector fragment of pUC19(d)-bSt and fragment 14 are ligated together and transformed into competent E. coli cells. This resultant vector is designated pUC19(d)-bSt-ala and can be used as the transition vector for the position 98 base changes following the steps described in Example 3A above.
Example 4. Biological Assay.

Samples of deamidated rbSt are treated for relative potency using the hypophysectomized (pituitary surgically removed) rat bioassay. Two hundred hypophysectomized female Sprague Dawley rats weighing between 75 and 150 grams are used in each experiment. They are fed ad libitum pelleted Purina Rat Chow which is a commercial product and watered with deionized and rechlorinated water. Room conditions are set at a temperature of 26.67°C (80°F) and relative humidity of 50%. Air exchange is approximately 20 exchanges per hour and the photoperiod is set at 12 hours light and 12 hours dark, with the light cycle commencing at approximately 6:00 a.m.
A. The rats are monitored for a 12-day preliminary period to allow for adaptation to environmental and feeding conditions. Body weights are obtained on four occasions between days one and twelve. Rats which show weight losses of one gram per day or less or weight gains of 2 grams or less during the preliminary period are selected for injection with bSt. Only data from rats which are completely hypophysectomized as demonstrated by post mortem evaluation will be included in the final analysis. The rats are ranked according to ascending magnitude of average daily weight gain (ADG) and seven blocks of 25 rats are created. Treatments within each block are randomly assigned.
B. bSt treatments are begun and last 10 days. During this time, test compounds are injected twice daily for nine days and body weights are monitored using a Mettler Model 3600 top loading balance equipped with lab-Pac programming which determines the weight of the animals while taking into account their movements.

Stock solutions of rbSt at 2 mg/ml are prepared in a buffer of 0.03M NaHCO₃ and 0.15M NaCl at pH 9.5. To facilitate suspension of rbSt, the lyophilized preparations are first dissolved in this buffer at pH 10.8, pH is then adjusted to 9.5 using 2N HCl and brought to final volume using the stock buffer at pH 9.5 and filtered if necessary.

The stock solutions are diluted using stock buffer (pH 9.5) to solutions of 37.5, 75, 150 and 300 mg of protein/ml. The experimental rats are injected subcutaneously twice daily with 100 µl of the respective solutions and controls receive 100 µl of buffer. The experiment lasts 10 days with average daily weight gain monitored.

A statistical analysis of the relative potency for the various test samples of rbSt is as follows: Untreated rbSt purified by HPLC was assigned a relative potency value of 1.00; the deamidated species of rbSt had a relative value of 2.40 (1.85-3.25). This value is significant at the 95% confidence level. A deamidated composition of rbSt having a pI of between 6.0-5.0 was tested against rbSt from pituitary glands, the relative potency of the acidic species was from 0 to 20% of the pituitary standard.

Example 5. To increase the growth rate in cows, young beef steers are selected for treatment. Recombinant deamidated bovine somatotropin is administered subcutaneously to each animal at a rate of 50 milligrams per day for 100 days. The animals are permitted to eat and drink at will.

Example 6. To increase the milk production in dairy cows, cows sixty days post-partum are selected for treatment. Each animal receives recombinant deamidated bovine somatotropin administered subcutaneously at a rate of 50 milligrams per day for 300 days. The animals are permitted to eat and drink at will.

Example 7. To increase the number of mammary parenchymal cells in dairy heifers, cows that are about 30 days before the expected onset of puberty are selected for treatment. Each animal receives recombinant deamidated bovine somatotropin administered subcutaneously at a rate of 50 milligrams per day for 100 days at which point the animals should be bred according to normal management practices. The animals are permitted to eat and drink at will.

FIGURE 1. The following nucleotide and amino acid sequence is from European Patent Application 75,444. It represents a hybrid bSt molecule and is useful herein to point out the relevent asparagine within the specific β turn region of interest. (See underlined residue 98.)

**1**

```
met phe pro ala met ser leu ser gly leu phe ala asn ala val
ATG TTC CCA GCT ATG TCT CTA TCT GGT CTA TTC GCT AAC GCT GTT
```

**20**

```
leu arg ala gln his leu his gln leu ala ala asp thr phe lys
CTT CGT GCT CAG CAT CTT CAT CAG CTG GCT GCT GAC ACC TTC AAA
```

**40**

```
glu phe glu arg thr tyr ile pro glu gly gln arg tyr ser ile
GAG TTT GAG CGC ACC TAC ATC CCG GAG GGA CAG AGA TAC TCC ATC
```

```
gln asn thr gln val ala phe cys phe ser glu thr ile pro ala
CAG AAC ACC CAG GTT GCC TTC TGC TTC TCT GAA ACC ATC CCG GCC
```

**60**

```
pro thr gly lys asn glu ala gln gln lys ser asp leu glu leu
CCC ACG GGC AAG GAT GAG GCC CAG CAG AAA TCA GAC TTG GAG CTG
```

**80**

```
leu arg ile ser leu leu leu ile gln ser trp leu gly pro leu
CTT CGC ATC TCA CTG CTC CTC ATC CAG TCG TGG CTT GGG CCC CTG
```

**100**

```
gln phe leu ser arg val phe thr asn ser leu val phe gly thr
CAG TTC CTC AGC AGA GTC TTC ACC AAC AGC TTG GTG TTT GGC ACC
```

```
ser asp arg val tyr glu lys leu lys asp leu glu glu gly ile
TCG GAC CGT GTC TAT GAG AAG CTG AAG GAC CTG GAG GAA GGC ATC
```

**120**

```
leu ala leu met arg glu leu glu asp gly thr pro arg ala gly
CTG GCC CTG ATG CGG GAG CTG GAA GAT GGC ACC CCC CGG GCT GGG
```

**140**

```
gln ile leu lys gln thr tyr asp lys phe asp thr asn met arg
CAG ATC CTC AAG CAG ACC TAT GAC AAA TTT GAC ACA AAC ATG CGC
```

**160**

```
ser asp asp ala leu leu lys asn tyr gly leu leu ser cys phe
AGT GAC GAC GCG CTG CTC AAG AAC TAC GGT CTG CTC TCC TGC TTC
```

```
arg lys asp leu his lys thr glu thr tyr leu arg val met lys
CGG AAG GAC CTG CAT AAG ACG GAG ACG TAC CTG AGG GTC ATG AAG
```

**180**                                        **190**

```
cys arg arg phe gly glu ala ser cys ala phe stop
TGC CGC CGC TTC GGG GAG GCC AGC TGC GCA TTC TAG
```

## FIGURE 2.

### Original Sequence of Bovine Somatotropin

```
                                       98
5' CTG.CAG.TTC.CTC.AGC.AGA.GTC.TTC.ACC.AAC.AGC.TTG.GTG 3'
                                       asn
```

### Synthetic Oligonucleotides

```
                                       98
5' CTG.CAG.TTC.CTC.AGC.AGA.GTC.TTC.ACC.GAC.AGC.TTG.GTG 3'
                                       asp
```

```
5' CTG.CAG.TTC.CTC.AGC.AGA.GTC.TTC.ACC.GAA.AGC.TTG.GTG 3'
                                       glu
```

CHART 1. Construction of pUC(d)

### pUC19

```
              BamHI  XbaI  HinII   PstI  HindIII
   *     ───────┴─────┴─────┴───────┴─────┴─────────   *
        │                                       │
        Amp                                     Ori
```

a) Plasmid pUC19 is cut with HindIII, treated with Klenow Enzyme and recut with HinII. The vector (linear) is isolated and ligated to form pUC19(d) with an approximate siye of 2.7 kb. The vector is maintained in E. coli.

### pUC19(d)

```
                   BamHI    XbaI
      *     ──────────┴───────┴─────────   *
           │                       │
           Amp                     Ori
```

### Key

Amp = Ampicillin resistance.
Ori = Origin of replication.

CHART 2. Construction of pUC19(d)-bSt
a) Plasmid pBGH 33 – 4 (5.2 kb) is cut with Xbal and BamHI to isolate the intact BGH gene (0.9 kb).

**Fragment 1**

b) Plasmid pUC19(d) from Chart 1 is cut with Xbal and BamHI to yield fragment 2 (2.7 kb).

**Fragment 2**

c) Fragments 1 and 2 are isolated and ligated using T4 DNA ligase to yield plasmid pUC19(d)-bSt.

Key
Amp = Ampicillin resistance.
Ori = Origin of replication.
$P_{Trp}$ = Tryptophan promoter.
B = rbSt cDNA.

CHART 3. Construction of pM13-bSt'
a) Plasmid pBGH 33 – 4 is cut with Pstl and BamHI and fragment 3 (700 kb) containing the 3' end of the bSt gene is isolated.

**Fragment 3**

b) Plasmid pM13 mp-19 RF (7.25 kb) is cut with Pstl and BamHI and fragment 4 (7.20 kb) is isolated.

c) Fragments 3 and 4 are ligated to yield pM13-bSt.

13

```
       BamHI        PstI
  *       |           |                                    *
  |_____|
    |    BBBBBBBBB      |         |
  LacZ'               P_LacZ'    Ori
```

**Key**

**B** - Bovine somatotropin.
**LacZ'** - α fragment of β-galactosidase.
**P_LacZ'** - Promoter for fragment of β-galactosidase.
**Ori** - Origin of replication.

CHART 4. Construction of pUC19(d)-bSt*

a) Single stranded DNA from M13-bSt grown in dam⁻ cells are hybridized with a synthetic oligonucleotide having an appropriate base change at the codon coding for asparagine 98 to insert aspartic acid.

$$\Delta$$
$$5'* \text{ GACGTCAAGGAGTCGTCTCAGAAGTGGGTGTCGAACCAC } *3'$$
$$.....\text{CTGCAGTTCCTCAGCAGAGTCTTCACCAACAGCTTGGTG}.....$$

b) The primer is extended beyond the BamHI site of the rbSt 3' end using Polk (Klenow fragment). the heteroduplex portion of the vector is excised using PstI and BamHI and isolated to yield fragment 5.

c) pUC19(d)bSt (Chart 2) is cut with PstI and BamHI to remove the 3' portion of the bSt cDNA. Fragment 6 (3.0 kb) containing the vector portion of pUc19 and the 5' portion of bSt is isolated.

```
   PstI              Xba                    BamHI
    |_____|_____|
    BBBBBBBBBBBBBBBBBB      |         |
                          Ori       Amp
```

d) Fragments 5 and 6 are ligated and the resulting heteroduplex is transformed into E. coli which forms two populations of homoduplexes. The homoduplex containing the appropriate modifaction at position 98 (aspartic acid) is selected by gel sequencing and isolated for expression. The vector is designated as pUC19(d)-bSt*.

```
           XbaI    PstI    BamHI
  5'*        |       |       |                    *3'
  |_____|
           BBBBBBBBBBBBBBBB    |       |
                    Δ        Amp     Ori
```

**Key**

**B** - rbSt cDNA.
**Δ** - Site of nucleotide change.
**Amp** - Ampicillin resistance.
**Ori** - Origin of replication.

CHART 5. Construction of pBGH33-4-"Glu98"

a) Plasmid pUC19(d)-bSt* is cut with Cbal and Bamhl to yield fragment 7 (1.0 kb) which contains the entire coding sequence for rbSt as modified at position 98.

**Fragment 7**

```
XbaI                                              BamHI
  |                                                 |
 |BBBBBBBBBBBBBBBBBBBBBBBBBBBBBBBBBBBBBBBB|
                          Δ
```

b) Expression vector pBGH33-4 is cut with BamHI and fragment 8 (4.8 kb) is isolated.

**Fragment 8**

```
BamHI                                          XbaI
  |                                             |
            |              |              |
           Ori            Amp          P_Trp
```

c) Fragments 7 and 8 are ligated to form the expression vector pBGH33-4"Glu98" (5.8 kb).

```
                                    XbaI        BamHI
 *                                    |           |            *
                                     |BBBBBBBBBBB
         |         |                P_Trp        Δ
        Ori       Amp
```

**Key**

Ori — Origin of replication.
Amp — Ampicillin resistance.
P_Trp — Tryptophan promoter.
B — rbSt cDNA.
Δ — Site of nucleotide change.

CHART 6. pUC8-bSt'

a) Plasmid pBGH33-4 (Chart 2) is cut with EcoRI and PstI to obtain fragment 9 (550 bp) shich is isolated by gel electrophoresis and electroelution.

**Fragment 9**

```
EcoRI         XbaI      PvuII       PstI
  |            |          |          |
  |          |BBBBBBBBBBBBBBBBBBBBBBBBBBBB|
 P_Trp
```

b) Plasmid pUC-8 is cut with EcoRI and PstI to obtain fragment 10 (2.7 kb) which is isolated by gel electrophoresis and electroelution.

**Fragment 10**

```
EcoRI                    .              PstI
  |                                       |
  |        |                    |         |
          Ori                  Amp
```

c) Fragments 9 and 10 are ligated to yield pUC8-bSt' (3.3 kb).

```
                EcoRI    XbaI     PvuII    PstI
       *     ┃————————————┃————————┃————————┃————————————┃     *
             ┃            ┃BBBBBBBBBBBBBBBBBBBBBBBB
             ┃            ┃                              ┃
            Ori          P_Trp                         Amp
```

**Key**

Amp - Ampicillin resistance.
Ori - Origin of replication.
P_Trp - Tryptophan promoter.
B - rbSt cDNA.

CHART 7. The Construction of pUC-bSt'ala.
   a) Plasmid pUC8-bSt' (Chart 6) is cut with Cbal and Pvull to yield fragment 11 which is purified by gel electrophoresis and isolated by electroelution.

### Fragment 11

```
       XbaI        EcoRI                    PstI        PvuII
       ┃————————————┃——————————————————————┃————————————┃
       ┃BBBBBB┃     ┃                       ┃   BBBBBBBBB ┃
        P_Trp       Ori                   Amp
```

b) Fragment 12 ( 72 bp), a double stranded oligonucleotide containing the 5′ portion of bSt and incorporating an alanine codon at position one, is artificially synthesized.

### Fragment 12

```
              XbaI                       PvuII
              ┃——————————————————————————┃
              ┃ABBBBBBBBBBBBBBBBBBBBBBBBB┃
```

c) Fragments 11 and 12 are ligated with T4 ligase to yield pUC8-bSt'ala.

```
                PstI     PvuII      XbaI      EcoRI
       *     ┃————————————┃————————┃————————┃————————————┃     *
             ┃  ┃    ┃    BBBBBBBBBBBBBBBBBBBBBA   ┃      ┃
             Ori  Amp                            P_Trp
```

**Key**

Amp - Ampicillin resistance.
Ori - Origin of replication.
B - Bovine somatotropin cDNA.
A - Alanine codon.
P_Trp - Tryptophan promoter.

CHART 8. Construction of pUC19(d)bSt-ala
   a) Plasmid pUC19(d)bSt (Chart 2) is cut with Xbal and Pstl and fragment 13 (2.7 kb) is gel isolated.

**Fragment 13**

```
PstI          BamHI                          XbaI
 |              |                              |
 |BBBBBBBBBBB        |          |              |
                    Amp        Ori
```

b) Plasmid pUC8-bSt'ala (Chart 7) is cut with XbaI and PstI to yield fragment 14 (300 bp) which is gel isolated.

**Fragment 14**

```
XbaI                          ·          PstI
 |_____|
 |ABBBBBBBBBBBBBBBBBBBBBBBBBBB|
```

c) Fragments 13 and 14 are ligated to yield pUC19(d)bst'ala (3.3 kb).

```
                  XbaI       PstI      BamHI
     *        ·     |          |          |          *
     _____
              |          |    ABBBBBBBBBBBBBBBBBBB
            Amp        Ori
```

**Key**

Amp – Ampicillin Resistance.
Ori – Origin of replication.
B – Bovine somatotropin cDNA.
A – Alanine codon.

## Claims

1. A method for enhancing the growth of animals, which comprises administering to the animals a composition which comprises a mixture of electrophoretic species of recombinant or pituitary bovine somatotropin, wherein the predominant species have isoelectric points of 7.5 to 6.5.

2. A method for increasing the quantity of mammary parenchymal cells in a female ruminant, which comprises administering to the ruminant, at a developmental stage from the onset of puberty until the first parturition, a composition as defined in claim 1.

3. A method according to claim 2, wherein the composition is administered until the first conception.

4. A method for increasing milk production in a female ruminant, which comprises administering to the ruminant a composition as defined in claim 1.

5. A method according to any preceding claim, wherein the animal is a species of bovine (which, if a female ruminant, is a dairy cow).

6. A process for increasing the proportion of electrophoretic species of bovine somatotropin having isoelectric points of 7.5 to 6.5, which comprises subjecting recombinant or pituitary bovine somatotropin to a mild alkaline treatment and/or heat.

7. A process according to claim 6, wherein the electrophoretic species having isoelectric points outside the range of 7.5 to 6.5 are substantially eliminated by chromatographic and/or electrophoretic techniques.

8. A process for modifying recombinant or pituitary mammalian somatotropin having an asparagine residue which corresponds to the asparagine located at residues 96 to 101 of the most closely-related native somatotropin, which comprises chemically deaminating the said asparagine residue by subjecting the protein to alkaline or acid treatment.

9. A process according to claim 8, wherein the protein is exposed to a carbonate buffer at an alkaline pH.

EP 0 263 206 B1

10. A process for modifying the cDNA encoding a recombinant or pituitary mammalian somatotropin including amino-acid residues corresponding to residues 96 to 101 of the most closely-related native somatotropin, which comprises subjecting the cDNA to genetic engineering such that it encodes a protein in which any of the said corresponding residues is replaced by a glutamic or aspartic acid residue.

11. A process according to claim 10, wherein the replaced residue corresponds to an asparagine residue.

12. A process according to any of claims 8 to 11, wherein the somatotropin is of bovine origin.

13. A process according to claim 12, wherein the somatotropin has an alanine at the amino terminal end.

14. A method for producing a recombinant or pituitary mammalian somatotropin including, instead of a neutrally-charged amino-acid residue correspond to one of residues 96 to 101 of the most closely-related native somatotropin, the residue of glutamic acid or aspartic acid, which comprises expressing a vector comprising DNA coding for the somatotropin.

**Patentansprüche**

1. Verfahren zum Beschleunigen des Wachstums von Tieren, welches das Verabreichen einer Zusammensetzung, welche eine Mischung einer elektrophoretischen Spezies von rekombinanten oder hypophysären Rinder-Somatropin enthält, umfaßt, worin die überwiegende Spezies isoelektrische Punkte von 6,5 bis 7,5 aufweist.

2. Verfahren zur Vergrößerung der Menge von Brustparenchymzellen bei einem weiblichen Wiederkäuer, welches das Verabreichen der Zusammensetzung nach Anspruch 1 an den Wiederkäuer im Entwicklungsstadium vom Einsetzen der Pubertät bis zum ersten Gebären umfaßt.

3. Verfahren nach Anspruch 2, worin die Zusammensetzung bis zur ersten Empfängnis verabreicht wird.

4. Verfahren zur Steigerung der Milchproduktion bei einem weiblichen Wiederkäuer, welches das Verabreichen einer Zusammensetzung nach Anspruch 1 an den Wiederkäuer umfaßt.

5. Verfahren nach einem der vorhergehenden Ansprüche, worin das Tier eine Rinderart darstellt (welche im Falle eines weiblichen Wiederkäuers eine Milchkuh ist.)

6. Verfahren zum Vergrößern des Verhältnisses der elektrophoretischen Spezies von Rinder-Somatotropin, welche isoelektrische Punkte von 7,5 bis 6,5 besitzt, welches Verfahren das Aussetzen des rekombinaten oder hypophysären Rinder-Somatotropins einer milden alkalischen Behandlung und/oder einer Erwärmung umfaßt.

7. Verfahren nach Anspruch 6, worin die elektrophoretischen Spezies, welche isoelektrische Punkte außerhalb des Bereiches von 7,5 bis 6,5 aufweisen, größtenteils durch chromatographische und/oder elektrophoretische Techniken entfernt werden.

8. Verfahren zum Modifizieren von rekombinanten oder hypophysären Säuger-Somatotropin, welches einen Asparaginrest besitzt, welcher dem Asparagin, welches an den Resten 96 bis 101 des am nächsten verwendeten nativen Somatotropins liegt entspricht, welches Verfahren die chemische Deamidierung des Asparaginrestes durch Aussetzen des Proteins einer alkalischen oder sauren Behandlung umfaßt.

9. Verfahren nach Anspruch 8, worin das Protein bei einem alkalischen pH-Wert einem Karbonatpuffer ausgesetzt wird.

10. Verfahren zum Modifizieren der cDNA, welche für ein rekombinantes oder hypophysäres Säuger-Somatotropin kodiert, welches Aminosäurereste, welche den Resten 96 bis 101 des am nächsten verwandten nativen Somatotropin entsprechen umfaßt, welches Verfahren das Aussetzen der cDNA einer Genmanipulation umfaßt, so daß es für ein Protein, in welchem jeder der entsprechenden Reste durch einen Glutamin- oder Asparaginsäurerest ersetzt ist kodiert.

11. Verfahren nach Anspruch 10, worin der ersetzte Rest einem Asparaginrest entspricht.

12. Verfahren nach einem der Ansprüche 8 bis 11, worin das Somatotropin von einem Rind stammt.

13. Verfahren nach Anspruch 12, worin das Somatotropin einen Alaninrest an dem Aminoende aufweist.

14. Verfahren zum Herstellen eines den Rest von Glutaminsäure und Asparaginsäure, an Stelle eines neutralen Aminosäurerestes, welcher einem der Reste 96 bis 101 der am nächsten verwandten nativen Somatotropins entspricht, enthaltenden rekombinanten oder hypophysären Säuger-Somatotropins welches Verfahren einen DNA enthaltenden Vektor ausdrückt, welcher für das Somatotropin kodiert.

**Revendications**

1. Procédé pour accroître la croissance d'animaux, qui consiste à administrer aux animaux une composition qui comprend un mélange de types électrophorétiques de somatotrophine bovine recombinante ou hypophysaire, dans lequel les types prédominants possèdent des points isoélectriques de 7,5 à 6,5.

2. Procédé pour accroître la quantité de cellules parenchymateuses mammaires chez un ruminant femelle, qui consiste à administrer au ruminant, à un stade de développement entre le début de la puberté et la première parturition, une composition suivant la revendication 1.

18

3. Procédé suivant la revendication 2, dans lequel la composition est administrée jusqu'à la première conception.

4. Procédé pour accroître la production de lait chez un ruminant femelle, qui consiste à administrer au ruminant une composition suivant la revendication 1.

5. Procédé suivant l'une quelconque des revendications précédentes, dans lequel l'animal est une espèce bovine (qui, s'il s'agit d'un ruminant femelle, est une vache laitière).

6. Procédé pour accroître la proportion de types électrophorétiques de somatotrophine bovine ayant des points isoélectriques de 7,5 à 6,5, qui consiste à soumettre de la somatotrophine bovine recombinante ou hypophysaire à un traitement alcalin doux et/ou à la chaleur.

7. Procédé suivant la revendication 6, dans lequel les types électrophorétiques ayant des points isoélectriques hors de l'intervalle de 7,5 à 6,5 sont éliminés en grande partie par des techniques chromatographiques et/ou électrophorétiques.

8. Procédé de midification de somatotrophine recombinante ou hypophysaire mammifère ayant un résidu asparagine qui correspond à l'asparagine située au niveau des résidus 96 à 101 de la somatotrophine native la plus étroitement apparentée, qui consiste à désamider chimiquement ledit résidu asparagine en soumettant la protéine à un traitement alcalin ou acide.

9. Procédé suivant la revendication 8, dans lequel la protéine est mise en contact avec un tampon au carbonate, à ph alcalin.

10. Procédé de modification de l'ADNc codant pour une somatotrophine ou hypophysaire de mammifère comprenant des résidus d'aminoacides correspondant aux résidus 96 à 101 de la somatotrophine native la plus étroitement apparentée, qui consiste à soumettre l'ADNc à des techniques de génie génétique de sorte qu'il code pour une protéine dans laquelle n'importe lequel des résidus correspondants est remplacé un résidu d'acide glutamique ou acide aspartique.

11. Procédé suivant la revendication 10, dans lequel le résidu remplacé correspond à un résidu asparagine.

12. Procédé suivant l'une quelconque des revendications 8 à 11, dans lequel la somatotrophine est d'origine bovine.

13. Procédé suivant la revendication 12, dans lequel la somatotrophine possède une alanine à l'extrémité amino-terminale.

14. Procédé de production d'une somatotrophine recombinante ou hypophysaire de mammifère comprenant, à la place d'un résidu aminoacide de charge neutre correspondant à l'un des résidus 96 à 101 de la somatotrophine native la plus étroitement apparentée, le résidu d'acide glutamique ou d'acide aspartique consiste à exprimer un vecteur comprenant de l'ADN codant pour la somatotrophine.